# EUROPEAN PATENT APPLICATION

(11) **EP 2 103 257 A1**
(43) Date of publication of application: **23.09.2009**
(21) Application number: 09003478.6
(22) Date of filing: 10.03.2009
(51) Int. Cl.: A61B 6/00, A61B 8/08, A61B 6/04

(54) **Medical imaging apparatus**

(30) Priority: 17.03.2008 JP 2008067080
(71) Applicant: Fujifilm Corporation, Tokyo 106-8620 (JP)
(72) Inventor: Yuji, Mikami, Ashigarakami-gun Kanagawa 258-8538 (JP); Sendai, Tomonari, Ashigarakami-gun Kanagawa 258-8538 (JP)
(74) Representative: Klunker . Schmitt-Nilson . Hirsch

(57) **Abstract**

In a medical imaging apparatus for imaging mammary gland and breast while compressing the breast with a compression plate, the influence of reflection and attenuation of ultrasonic waves in the compression plate can be reduced to improve the image quality of ultrasonic images. The medical imaging apparatus includes: an imaging stage 19; a compression plate 13 for compressing an object between the imaging stage 19 and itself; an ultrasonic probe 16 provided to maintain acoustic connection with the compression plate 13, for transmitting ultrasonic waves according to drive signals and receiving ultrasonic echoes to output reception signals; an ultrasonic imaging unit 42-46 for supplying the drive signals to the ultrasonic probe 16 and generating image data based on the reception signals; and a control unit 41 for controlling the ultrasonic imaging unit 42-46 according to information based on a material and/or a thickness of the compression plate 13 to adjust levels of the drive signals.

## Description

### BACKGROUND OF THE INVENTION

### Field of the Invention

The present invention relates to a medical imaging apparatus for imaging mammary gland and breast while compressing the breast with a compression plate for diagnoses of breast cancer.

### Description of a Related Art

Conventionally, an imaging method using radiation (X-ray, α-ray, β-ray, γ-ray, electron ray, ultraviolet ray, or the like) is utilized in various fields, and particularly, in the medical field, the method is one of the most important means for diagnoses. Radiation images obtained by X-ray imaging (X-ray mammography) of breasts for breast cancer diagnoses are useful for finding calcification as a precursor of mass and cancer, but finding calcification may be difficult depending on mammary gland density or the like of an object to be inspected. Accordingly, using radiation and ultrasonic wave in combination to make diagnoses based on both radiation images and ultrasonic images has been studied. X-ray mammography and ultrasonic imaging have the following features, respectively.

X-ray mammography is suitable for exposing calcification as one of early symptoms of a cancer, and enables detection with high sensitivity and high resolving power. Especially, in the case where mammary gland tissues have become atrophied and replaced with fat (so-called "fat breast") as is the case of postmenopausal women, more information can be obtained by X-ray mammography. However, the X-ray imaging has a disadvantage that detection capability of specific natures of tissues (tissue properties) is low.

Further, in an X-ray image, mammary glands are expressed in homogeneous soft tissue density, and thus, the detection of tumor mass is difficult for the case where mammary glands have developed (so-called, "dense breast") as is the case of adolescent to premenopausal women. Furthermore, in X-ray mammography, only two-dimensional images in which an object to be inspected as a solid is projected on a plane can be obtained. On this account, even when a tumor mass is found, it is difficult to grasp information on the location in the depth direction, the size, and so on of the tumor mass.

On the other hand, in ultrasonic imaging, specific natures of tissues (e.g., the difference between a cystic tumor and a solid matter) can be detected and also a lobular cancer can be detected. Further, real time observation of images and three-dimensional image generation are possible. However, accuracy of ultrasonic imaging examination often depends on the skill of an operator such as a doctor, and reproducibility is low. Further, it is difficult to observe minute calcification in an ultrasonic image.

As described above, each of the X-ray mammography examination and the ultrasonic imaging examination has both merits and demerits, and it is desirable that both examinations are performed for reliably finding breast cancer. Since the X-ray mammography examination is performed while the object (breast) is compressed by a compression plate, in order to make diagnoses based on X-ray images and ultrasonic images of the object in the same condition, the ultrasonic imaging examination is necessary to be performed in the same condition as that when the X-ray mammography examination is performed, that is, while the object (breast) is compressed by the compression plate. Accordingly, a medical imaging apparatus for imaging mammary gland and breast by using radiation and ultrasonic waves in combination is considered.

In the medical imaging apparatus, ultrasonic waves transmitted from ultrasonic transducers provided close to the compression plate pass through the compression plate and reach a breast, and ultrasonic echoes reflected by the breast pass through the compression plate again and are received by the ultrasonic transducers. However, when ultrasonic waves pass through the compression plate, intensity of the ultrasonic waves reaching the breast becomes lower due to influence of reflection and attenuation, and image quality is deteriorated in the ultrasonic images.

As a related technology, U.S. Patent US-A-5479927 discloses, in a medical imaging apparatus for imaging mammary gland and breast by using radiation and ultrasonic waves in combination, polymethylpentene advantageous in radiation transmissivity and ultrasonic transmissivity or the like is selected as a material of a compression plate. However, if a suitable material is selected as the material of the compression plate, only the reflection and attenuation of ultrasonic waves can be reduced, and further improvements are necessary to obtain good image quality in ultrasonic images.

### SUMMARY OF THE INVENTION

Accordingly, in view of the above-mentioned points, a purpose of the present invention is, in a medical imaging apparatus for imaging mammary gland and breast while compressing the breast with a compression plate, to further reduce the influence of reflection and attenuation of ultrasonic waves in the compression plate to improve the image quality of ultrasonic images.

In order to accomplish the above-mentioned purpose, a medical imaging apparatus according to one aspect of the present invention includes: an imaging stage on which an object to be inspected is mounted; a compression plate having a first surface for compressing the object and a second surface opposed to the first surface, for compressing the object between the imaging stage and itself; an ultrasonic probe provided to maintain acoustic connection with the second surface of the compression plate, for transmitting ultrasonic waves according to drive signals and receiving ultrasonic echoes generated when the transmitted ultrasonic waves are reflected by the object to output reception signals; ultrasonic imaging means for supplying the drive signals to the ultrasonic probe and generating image data based on the reception signals outputted from the ultrasonic probe; and control means for controlling the ultrasonic imaging means according to information based on a material and/or a thickness of the compression plate to adjust levels of the drive signals to be supplied to the ultrasonic probe.

According to the one aspect of the present invention, since the reception intensity of ultrasonic echoes rises by adjusting the ultrasonic output according to the information based on the material and/or thickness of the compression plate, the influence of reflection and attenuation of ultrasonic waves in the compression plate can be reduced to improve the image quality of ultrasonic images.

### BRIEF DESCRIPTION OF THE DRAWINGS

Fig. 1 is a block diagram showing a configuration of a medical imaging apparatus according to the first embodiment of the present invention;
Fig. 2 is a side view showing an appearance of the imaging unit of the medical imaging apparatus shown in Fig. 1;
Fig. 3 is a perspective view showing the appearance of the imaging unit of the medical imaging apparatus shown in Fig. 1;
Fig. 4 shows a difference between attenuation amounts of ultrasonic waves according to types of compression plates;
Fig. 5 is a block diagram showing a configuration of a medical imaging apparatus according to the second embodiment of the present invention;
Fig. 6 shows an example of an ultrasonic image;
Fig. 7 is a block diagram showing a configuration of a medical imaging apparatus according to the third embodiment of the present invention;
Fig. 8 shows an ultrasonic detector used in the third embodiment of the present invention and a part around the detector;
Fig. 9 shows an ultrasonic detector used in the first modified example of the third embodiment of the present invention and a part around the detector; and
Fig. 10 shows an ultrasonic detector used in the second modified example of the third embodiment of the present invention and a part around the detector.

### DESCRIPTION OF THE PREFERRED EMBODIMENTS

Hereinafter, preferred embodiments of the present invention will be explained in detail with reference to the drawings. The same reference numbers are assigned to the same component elements and the description thereof will be omitted.

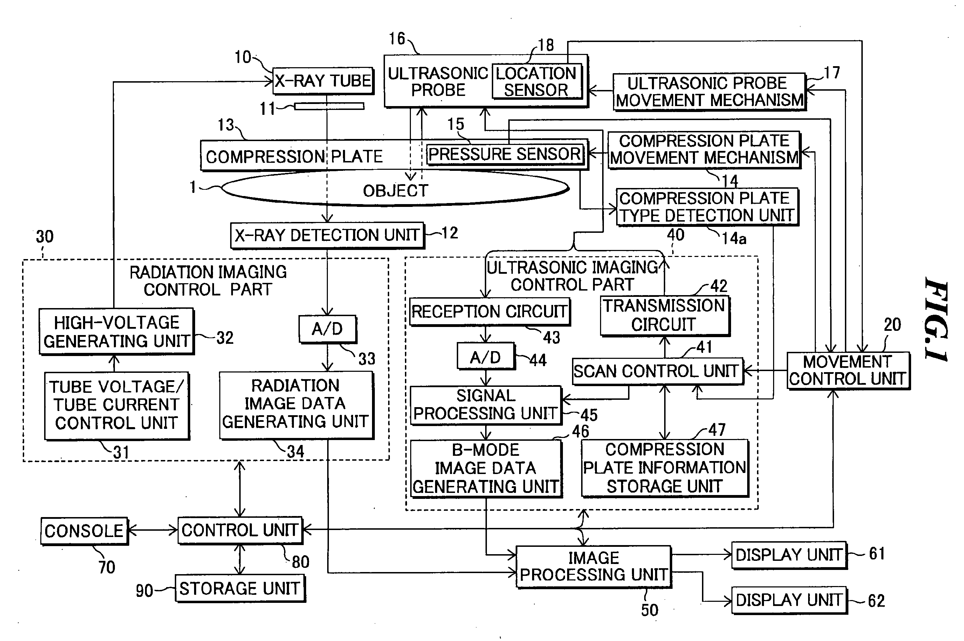

Fig. 1 is a block diagram showing a configuration of a medical imaging apparatus according to the first embodiment of the present invention. The medical imaging apparatus is a medical imaging apparatus having both a function of a radiation mammography apparatus for applying radiation to a breast, detecting the radiation transmitted through the breast, and thereby, generating a radiation image, and a function of an ultrasonic diagnostic apparatus for transmitting ultrasonic waves to the breast, receiving ultrasonic echoes reflected within the breast, and thereby, generating ultrasonic images. As below, the case of using an X-ray as radiation will be explained, however, α-ray, β-ray, γ-ray, electron ray, ultraviolet ray, or the like may be used.

As shown in Fig. 1, the medical imaging apparatus has an X-ray tube 10, a filter 11, an X-ray detection unit 12 for detecting an X-ray generated by the X-ray tube 10 and transmitted through an object to be inspected 1, a compression plate 13 for pressing a breast as the object, a compression plate movement mechanism 14 for moving the compression plate 13, a compression plate type detection unit 14a for detecting the type of the compression plate 13, a pressure sensor 15 for detecting pressure applied to the compression plate 13, an ultrasonic probe 16 including plural ultrasonic transducers for transmitting and receiving ultrasonic waves, a probe movement mechanism 17 for moving the ultrasonic probe 16, and a location sensor 18 for detecting the location of the ultrasonic probe 16 in an imaging unit.

Further, the medical imaging apparatus has a movement control unit 20 for controlling the compression plate movement mechanism 14, the probe movement mechanism 17, and so on, a radiation imaging control part 30, an ultrasonic imaging control part 40, an image processing unit 50, display units 61 and 62, a console 70, a control unit 80, and a storage unit 90.

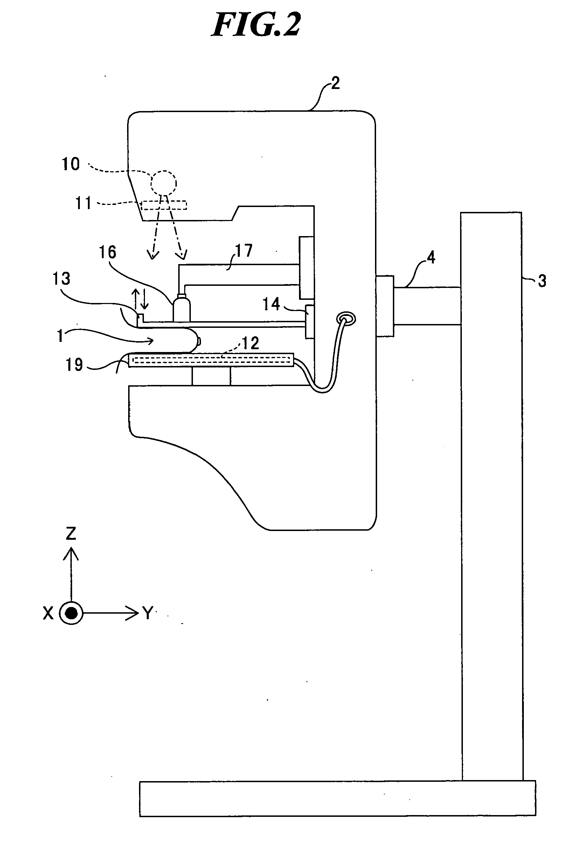

Fig. 2 is a side view showing an appearance of the imaging unit of the medical imaging apparatus shown in Fig. 1. As shown in Fig. 2, the imaging unit of the medical imaging apparatus has an arm part 2, a support 3 for movably holding the arm part 2 in the vertical direction (Z-axis direction), and a shaft part 4 for connecting the arm part 2 to the support 3. The arm part 2 is provided with the X-ray tube 10, the filter 11, the X-ray detection unit 12, an imaging stage 19 on which an object 1 to be inspected is mounted, the compression plate 13 for pressing the object 1 between the imaging stage 19 and itself, the compression plate movement mechanism 14 for moving the compression plate 13, the ultrasonic probe 16, and the probe movement mechanism 17 for moving the ultrasonic probe 16 in the X-axis, Y-axis, and Z-axis directions. Here, the X-ray tube 10 and the filter 11 form a radiation emitting unit.

The X-ray tube 10 emits an X-ray when a tube voltage is applied thereto. The filter 11 is made of a material such as molybdenum (Mo) or rhodium (Rh), and selectively transmits a desired wavelength component of plural wavelength components contained in the X-ray emitted by the X-ray tube 10. The X-ray detection unit 12 is a flat panel X-ray detector (FPD) for imaging an X-ray image by detecting the X-ray transmitted through the object 1 at plural detection points in a two-dimensional region. The X-ray radiated from the X-ray tube 10 and transmitted through the object 1 is applied to the respective detection points, and thereby, detection signals having magnitudes corresponding to the intensity of the X-ray are outputted from the X-ray detection unit 12. The detection signals are inputted via a cable to the X-ray imaging control unit 30 (Fig. 1).

The compression plate 13 has a compression surface (the lower surface in Fig. 2) for compressing the object 1 along the radiation direction of the X-ray and a surface opposite to the compression surface (the upper surface in Fig. 2). The compression plate 13 is provided in parallel to the imaging stage 19, and the compression plate movement mechanism 14 moves the compressionplate 13 nearly in the vertical direction (Z-axis direction) under the control of the movement control unit 20 (Fig. 1) . The pressure sensor 15 (Fig. 1) detects the pressure applied to the compression plate 13 and the movement control unit 20 controls the compression plate movement mechanism 14 based on the detection result. The object (breast) 1 is sandwiched by the compression plate 13 and the imaging stage 19, and X-ray imaging and ultrasonic imaging are performed with the homogeneous thickness of the breast.

Here, the compression plate 13 is necessary to be optically transparent for positioning when the breast is compressed or confirmation of the compression state, and desirably formed of a material that transmits the X-ray radiated from the X-ray tube 10 and easily propagates ultrasonic waves to be transmitted from the ultrasonic probe 16. As a material of the compression plate 13, a resin such as polycarbonate, acryl, or polymethylpentene disclosed in US-A-5479927 having suitable values in acoustic impedance that affects the reflectance of ultrasonic waves and attenuation coefficient that affects the attenuation of ultrasonic waves may be used, for example. As the compression plate 13, plates in plural sizes and shapes are prepared for uses, and the thickness of the compression plate 13 is about 2mm to 4mm. The reflectance and/or attenuation coefficient changes depending on the material and/or thickness of the compression plate 13.

The ultrasonic probe 16 includes one-dimensionally or two-dimensionally arranged plural ultrasonic transducers. Each ultrasonic transducer transmits ultrasonic waves to the object based on the applied drive signal, and receives ultrasonic echoes and outputs a reception signal.

Each ultrasonic transducer is configured by a vibrator in which electrodes are formed on both ends of a material havingapiezoelectricproperty (piezoelectricmaterial) such as a piezoelectric ceramic represented by PZT (Pb (lead) zirconate titanate), a polymeric piezoelectric element represented by PVDF (polyvinylidene difluoride), or the like. When a voltage is applied to the electrodes of the vibrator by transmitting pulsed or continuous wave electric signals, the piezoelectric material expands and contracts. By the expansion and contraction, pulsed or continuous wave ultrasonic waves are generated from the respective vibrators, and an ultrasonic beam is formed by synthesizing these ultrasonic waves. Further, the respective vibrators expand and contract by receiving propagating ultrasonic waves and generate electric signals. These electric signals are outputted as reception signals of the ultrasonic waves, and inputted to the ultrasonic imaging control part 40 (Fig. 1) via a cable.

The ultrasonic probe 16 may be moved in close contact with the compression plate 13 or moved apart from the compression plate 13 with an ultrasonic transmission medium such as echo gel inserted between the compression plate 13 and itself. It is more preferable that an ultrasonic transmission medium such as echo gel is applied to the compression plate 13, and the ultrasonic probe 16 is moved in contact with the compressionplate13. Further, an operator may move the ultrasonic probe 16, or the probe movement mechanism 17 may move the ultrasonic probe 16. As below, the latter case will be explained.

The location of the ultrasonic probe 16 is detected by the location sensor 18 provided in the ultrasonic probe 16. The movement control unit 20 (Fig. 1) grasps the location of the ultrasonic probe 16 based on the output signal of the location sensor 18 and controls the probe movement mechanism 17. The ultrasonic probe 16 transmits and receives ultrasonic waves while the probe movement mechanism 17 moves the ultrasonic probe 16, and thereby, ultrasonic imaging is performed.

Referring to Fig. 1 again, a radiation imaging system will be explained.

The radiation imaging control part 30 includes a tube voltage/tube current control unit 31, a high-voltage generating unit 32, an A/D converter 33, and a radiation image data generating unit 34.

In the X-ray tube 10, the X-ray transparency is determined according to the tube voltage applied between the cathode and the anode, and the amount of X-ray emission is determined according to the time integration of the tube current flowing between the cathode and the anode. The tube voltage/tube current control unit 31 adjusts imaging conditions of the tube voltage, tube current, and so on according to target values. The target values of the tube voltage and the tube current may be manually adjusted by the operator using the console 70. The high-voltage generating unit 32 generates a high voltage to be applied to the X-ray tube 10 under the control of the tube voltage/tube current control unit 31. The A/D converter 33 converts analog radiation detection signals outputted from the X-ray detection unit 12 into digital signals (radiation detection data), and the radiation image data generating unit 34 generates radiation image data based on the radiation detection data.

Next, an ultrasonic imaging system will be explained.

The ultrasonic imaging control part 40 includes a scan control unit 41, a transmission circuit 42, a reception circuit 43, an A/D converter 44, a signal processing unit 45, a B-mode image data generating unit 46, and a compression plate information storage unit 47. Here, the transmission circuit 42 to B-mode image data generating unit 46 form ultrasonic imaging means.

The scan control unit 41 sets frequencies and voltages of the drive signals to be applied from the transmission circuit 42 to the respective ultrasonic transducers of the ultrasonic probe 16 and adjusts the frequency and sound pressure of the ultrasonic waves to be transmitted under the control of the movement control unit 20. Further, the scan control unit 41 has a transmission control function of transmission directions of ultrasonic beams and selecting transmission delay patterns according to the set transmission directions, and a reception control function of sequentially setting reception directions of ultrasonic echoes and selecting reception delay patterns according to the set reception directions.

Here, the transmission delay pattern refers to a delay time pattern to be provided to the plural drive signals for forming an ultrasonic beam in a desired direction with the ultrasonic waves transmitted from the plural ultrasonic transducers of the ultrasonic probe 16, and the reception delay pattern refers to a delay time pattern to be provided to the plural reception signals for extracting ultrasonic echoes from the desired direction with the ultrasonic waves received by the plural ultrasonic transducers. Plural transmission delay patterns and reception delay patterns are stored in a memory or the like.

The transmission circuit 42 generates plural drive signals to be respectively applied to the plural ultrasonic transducers. In this regard, the transmission circuit 42 may adjust the amounts of delay of the plural drive signals and supply the signals to the ultrasonic probe 16 so that the ultrasonic waves to be transmitted from the plural ultrasonic transducers form an ultrasonic beam, or may supply plural drive signals to the ultrasonic probe 16 so that the ultrasonic waves to be transmitted at once from the plural ultrasonic transducers reach the entire imaging region of the object.

The reception circuit 43 amplifies the plural ultrasonic reception signals respectively outputted from the plural ultrasonic transducers, and the A/D converter 44 converts the analog ultrasonic reception signals amplified by the reception circuit 43 into digital ultrasonic reception signals. The signal processing unit 45 performs reception focus processing by providing the respective delay times to the plural ultrasonic reception signals based on the reception delay pattern selected by the scan control unit 41, and adding those ultrasonic reception signals. Through the reception focus processing, sound ray signals in which the focal point of the ultrasonic echoes is narrowed is formed.

Furthermore, the signal processing unit 45 corrects attenuation of the sound ray signals by distance according to the depths of the reflection positions of ultrasonic waves through STC (Sensitivity Time gain Control), and then, performs envelope detection processing with a low-pass filter or the like thereon to generate envelope signals.

The B-mode image data generating unit 46 performs processing such as logarithmic compression and gain adjustment on the envelope signals to generate image data, and converts (raster-converts) the image data into image data that follows the normal scan system of television signals to generate B-mode image data.

In the embodiment, the compression plate information storage unit 47 including a hard disk, memory, or the like stores correction amounts of ultrasonic outputs corresponding to attenuation amounts of ultrasonic waves measured or calculated based on materials and/or thicknesses of compression plates of usable types in the medical imaging apparatus. As specifically explained later, the ultrasonic outputs is adjusted based on the correction amounts stored in the compression plate information storage unit 47 for correction of the reduction in levels of ultrasonic waves due to the compression plate 13.

The image processing unit 50 performs necessary image processing such as gradation process on the radiation image data outputted from the radiation imaging control part 30 and the radiation image data outputted from the ultrasonic imaging control part 40 to generate image data for display. Thereby, radiation image and ultrasonic images are displayed on the display units 61 and 62, respectively.

The console 70 is used by the operator to operate the medical imaging apparatus. The control unit 80 controls the respective parts based on the operation of the operator. So far, the movement control unit 20, the radiation image data generating unit 34, the scan control unit 41, the signal processing unit 45, the B-mode image data generating unit 46, the image processing unit 50, and the control unit 80 are configured by a central processing unit (CPU) and software for allowing the CPU to execute various kinds of processing, however, they may be configured by a digital circuit or analog circuit. The software (program) is stored in the storage unit 90 formed by a hard disk, memory, or the like. Further, the transmission delay patterns and the reception delay patterns to be selected by the scan control unit 41 may be stored in the storage unit 90, or the storage unit 90 also may have the function of the compression plate information storage unit 47.

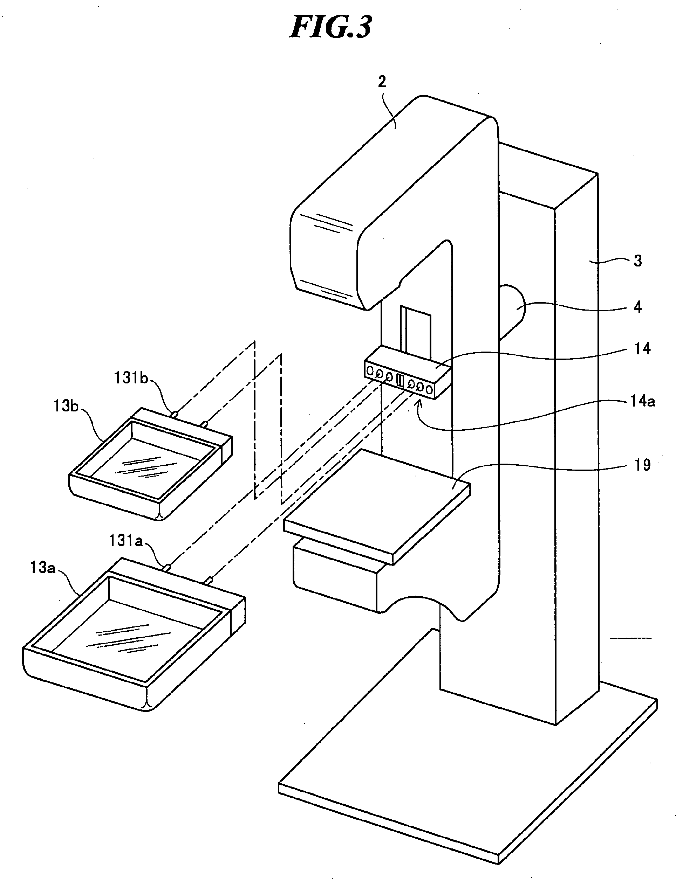

Fig. 3 is a perspective view showing an appearance of the imaging unit of the medical imaging apparatus shown in Fig. 1. In this medical imaging apparatus, plural types of compression plates may be replaced and used, and two types of compression plates 13a and 13b are shown in Fig. 3. For example, the compression plate 13a has a size corresponding to an imaging area of 24cmx30cm, and the compression plate 13b has a size corresponding to an imaging area of 18cm×24cm. Further, the compression plate 13a has two attachment projections 131a at a first interval and the compression plate 13b has two attachment projections 131b at a second interval. As described above, positions (intervals) of attachment projections are different depending on the types of compression plates, and the compression plate type detection unit 14a provided in the compression plate movement mechanism 14 can detect the type of the compression plate when the compression plate is actually attached to the compression plate movement mechanism 14.

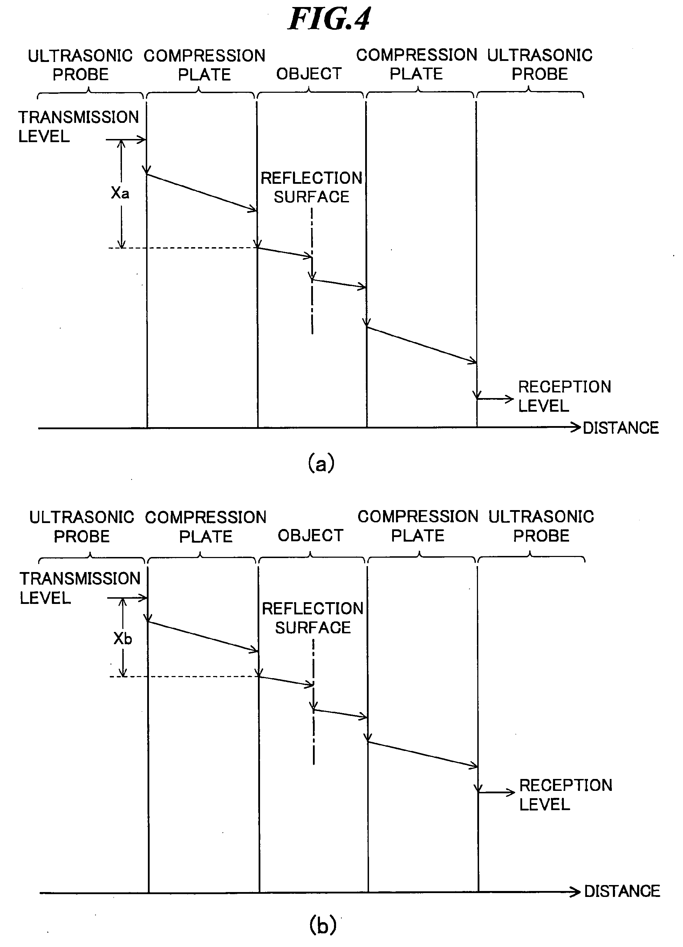

Fig. 4 shows a difference between attenuation amounts of ultrasonic waves according to the types of compression plates. Fig. 4 (a) shows an attenuation amount of ultrasonic waves when the compression plate 13a is used, and Fig. 4(b) shows an attenuation amount of ultrasonic waves when the compression plate 13b is used.

In Fig. 4(a), the ultrasonic waves transmitted at a predetermined transmission level from the ultrasonic probe is partially reflected at an interface between the ultrasonic probe and the compression plate, the ultrasonic waves entering the compression plate attenuate during propagation in the compression plate, and further, the ultrasonic waves transmitted through the compression plate is partially reflected at an interface between the compression plate and the object. As a result, the ultrasonic waves entering the object attenuate by an attenuation amount Xa relative to the ultrasonic waves transmitted from the ultrasonic probe. The ultrasonic waves entering the object propagate while attenuating, and is reflected on a reflection surface within the object and received by the ultrasonic probe via the compression plate.

The same phenomenon appears in Fig. 4 (b), however, an attenuation amount Xb when the compression plate 13b is used is smaller than the attenuation amount Xa when the compression plate 13a is used because the materials and/or thicknesses of the compression plates are different. The higher the level of the ultrasonic waves entering the object, the higher the S/N ratio of the obtained ultrasonic image. In consideration of the influence on the human body as the object, there is an appropriate value of the level of the ultrasonic waves entering the object. Accordingly, in the embodiment, attenuation amounts of ultrasonic waves when various types of compression plates are used are obtained by measurement or calculation in advance, and ultrasonic outputs are corrected so that the level of the ultrasonic waves entering the object becomes the appropriate value.

The compression plate information storage unit 47 shown in Fig. 1 stores correction amounts of ultrasonic outputs for the attenuation amounts of ultrasonic waves measured or calculated based on the materials and/or thicknesses of the respective compression plates. The scan control unit 41 searches the compression plate information storage unit 47 based on the type of the compression plate detected by the compression plate type detection unit 14a, and thereby, can load a correction amount corresponding to the detected type of compression plate. The scan control unit 41 controls the transmission circuit 42 according to the correction amount loaded from the compression plate information storage unit 47 to adjust the levels of the drive signals supplied to the ultrasonic probe 16. Thereby, the level of ultrasonic waves entering the object is maintained in an appropriate range.

Next, the second embodiment of the present invention will be explained.

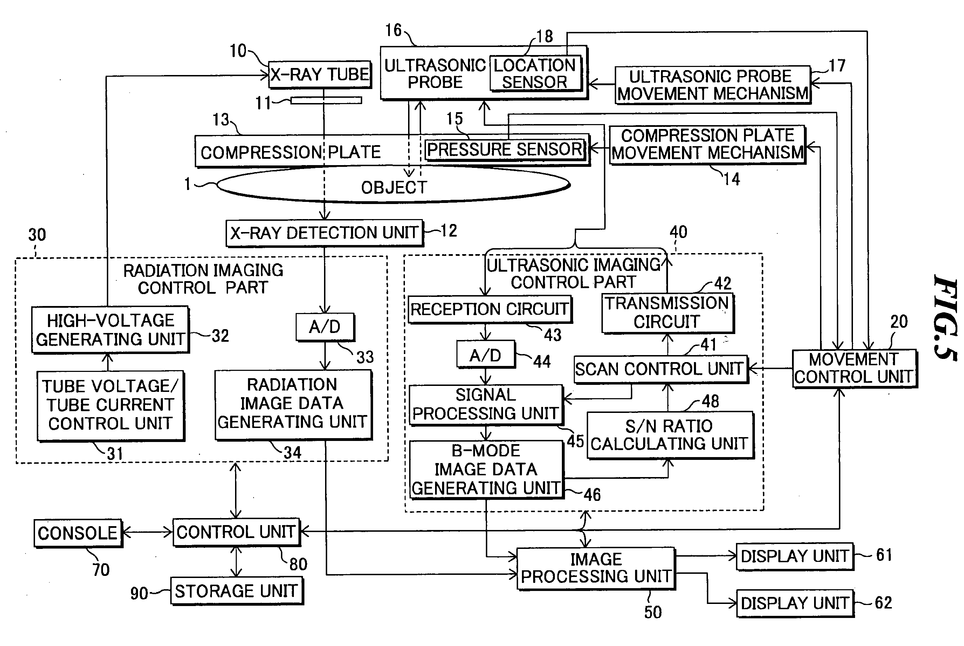

Fig. 5 is a block diagram showing a configuration of a medical imaging apparatus according to the second embodiment of the present invention. In the second embodiment, in order to correct the reduction of the level of ultrasonic waves due to the compression plate 13, the ultrasonic output is adjusted according to information based on the reception signals or image signals from the object in a predetermined position. As the information based on the reception signals or image signals, the level of reception signals, the brightness level represented by image data, the S/N ratio of ultrasonic images, or the like may be used. As below, the case of using the S/N ratio of ultrasonic images or the like will be explained.

In the second embodiment shown in Fig. 5, an S/N ratio calculating unit 48 is provided in place of the compression plate type detection unit 14a and the compression plate information storage unit 47 in the first embodiment shown in Fig. 1. The S/N ratio calculating unit 48 inputs B-mode image data generated by the B-mode image data generating unit 46, calculates an S/N ratio of an image based on image data representing the image of the object in the predetermined position (depth), and outputs information representing the calculated S/N ratio.

The scan control unit 41 controls the transmission circuit 42 according to information outputted from the S/N ratio calculating unit 48 to adjust the levels of the drive signals supplied to the ultrasonic probe 16. For example, the scan control unit 41 controls the transmission circuit 42 so that the S/N ratio calculated by the S/N ratio calculating unit 48 falls within a predetermined range. Thereby, the S/N ratio of the ultrasonic image is maintained in an appropriate range.

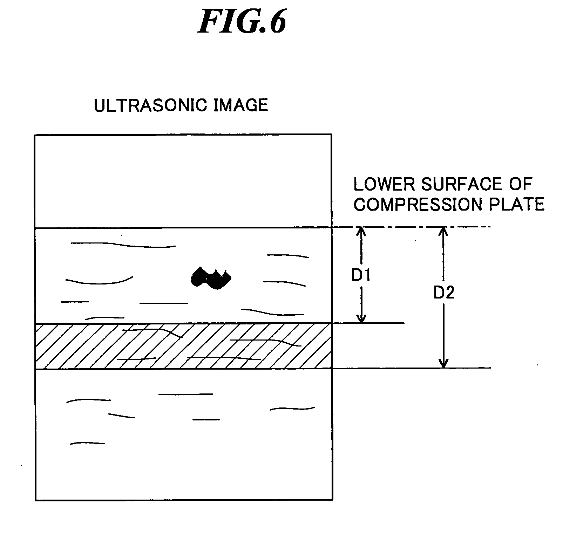

Here, the predetermined position (depth) where the S/N ratio is calculatedmaybe determined according to the distance from the compression plate 13. For example, in an ultrasonic image shown in Fig. 6, the predetermined position where the S/N ratio is calculated is determined within a range in which the distance from the lower surface of the compression plate 13 is D1 to D2. Alternatively, the predetermined position where the S/N ratio is calculated may be determined based on the thickness of the object compressed by the compression plate 13 (compressed thickness). For example, the predetermined position where the S/N ratio is calculated is determined in a position at the distance from the lower surface of the compression plate 13 of 30% of the compressed thickness.

Next, the third embodiment of the present invention will be explained.

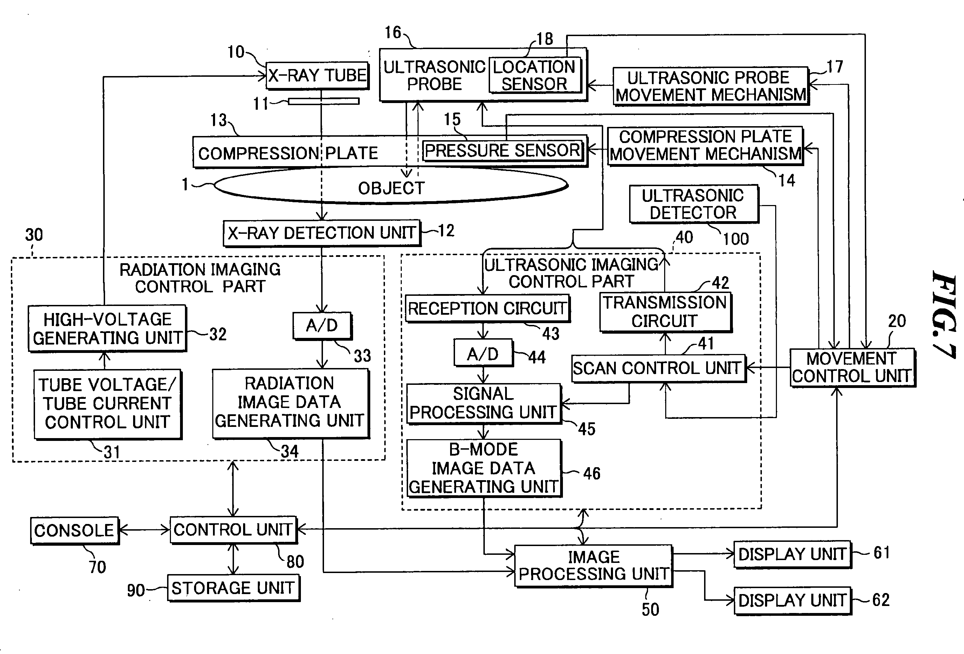

Fig. 7 is a block diagram showing a configuration of a medical imaging apparatus according to the third embodiment of the present invention. In the third embodiment, an ultrasonic detector 100 for detecting ultrasonic waves transmitted from the ultrasonic probe 16 is provided in place of the compression plate type detection unit 14a and the compression plate information storage unit 47 in the first embodiment shown in Fig. 1. In order to correct the reduction of the level of ultrasonic waves due to the compression plate 13, the ultrasonic output is adjusted according to the detection result of the ultrasonic detector 100.

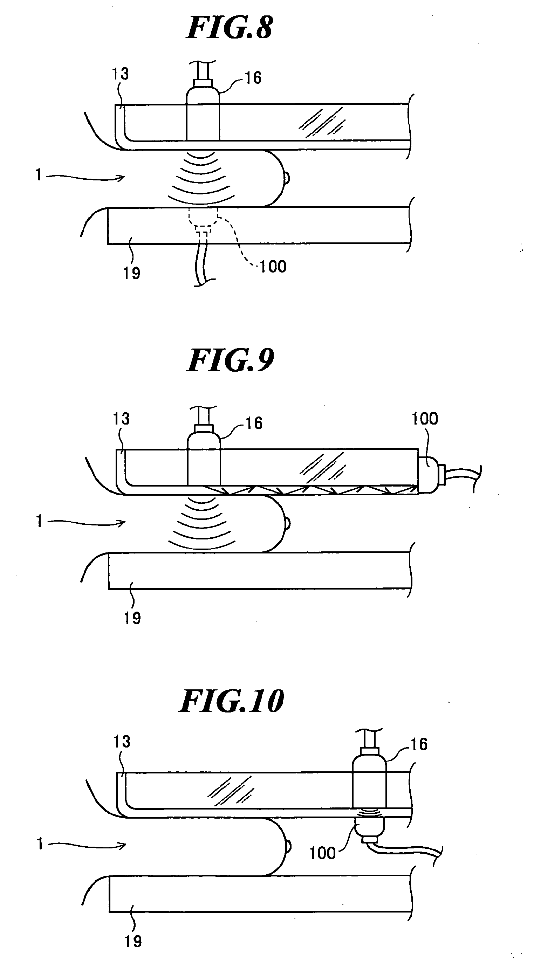

Fig. 8 shows the ultrasonic detector used in the third embodiment of the present invention and a part around the detector. The ultrasonic detector 100 is provided in a position facing the lower surface of the compression plate 13 on the imaging stage 19, and detects the ultrasonic waves transmitted from the ultrasonic probe 16 and passing through the object 1 and outputs a digital signal representing the level of ultrasonic waves as a detection result.

The scan control unit 41 shown in Fig. 7 controls the transmission circuit 42 according to the digital signal outputted from the ultrasonic detector 100 to adjust the levels of the drive signals supplied to the ultrasonic probe 16. For example, the scan control unit 41 controls the transmission circuit 42 so that the level of the ultrasonic waves detected by the ultrasonic detector 100 falls within a predetermined range. Thereby, the level of the ultrasonic waves entering the object is maintained in an appropriate range.

As shown in Fig. 8, when X-ray imaging is performed with the ultrasonic detector 100 remaining located in the position facing the lower surface of the compression plate 13, an image of the ultrasonic detector 100 appears in the X-ray image.
Accordingly, it is necessary to retract the ultrasonic detector 100 to the outside of the X-ray imaging range when X-ray imaging is performed or correct the X-ray image in which the image of the ultrasonic detector 100 appears to erase the image of the ultrasonic detector 100.

Next, a first modified example of the third embodiment of the present invention will be explained.

Fig. 9 shows an ultrasonic detector used in the first modified example of the third embodiment of the present invention and a part around the detector. The ultrasonic detector 100 is provided near the compression plate 13 to maintain the acoustic connection with the compression plate 13, and detects ultrasonic waves transmitted from the ultrasonic probe 16 and propagating while being reflected in the compression plate 13 and outputs a digital signal representing the level of the ultrasonic waves as a detection result.

The scan control unit 41 shown in Fig. 7 controls the transmission circuit 42 according to the digital signal outputted from the ultrasonic detector 100 to adjust the levels of the drive signals supplied to the ultrasonic probe 16. For example, the scan control unit 41 controls the transmission circuit 42 so that the level of the ultrasonic waves detected by the ultrasonic detector 100 falls within a predetermined range. Thereby, the level of the ultrasonic waves entering the object is maintained in an appropriate range.

Next, a second modified example of the third embodiment of the present invention will be explained.

Fig. 10 shows an ultrasonic detector used in the second modified example of the third embodiment of the present invention and a part around the detector. The ultrasonic detector 100 is provided to maintain the acoustic connection with the compression plate 13 on the lower surface of the compression plate 13, and detects ultrasonic waves transmitted from the ultrasonic probe 16 and propagating while being reflected in the compression plate 13 and outputs a digital signal representing the level of the ultrasonic waves as a detection result.

The scan control unit 41 shown in Fig. 7 controls the transmission circuit 42 according to the digital signal outputted from the ultrasonic detector 100 to adjust the levels of the drive signals supplied to the ultrasonic probe 16. For example, the scan control unit 41 controls the transmission circuit 42 so that the level of the ultrasonic waves detected by the ultrasonic detector 100 falls within a predetermined range. Thereby, the level of the ultrasonic waves entering the object is maintained in an appropriate range.

In the modified example, when the compression plate 13 is replaced and calibration is needed, it is desirable that the ultrasonic detector 100 is provided outside of the X-ray imaging range and ultrasonic imaging range, the ultrasonic probe 16 is moved above the ultrasonic detector 100, and the level of ultrasonic waves is measured.

## Claims

1. A medical imaging apparatus comprising:
an imaging stage (19) on which an object to be inspected is mounted;
a compression plate (13) having a first surface for compressing the object and a second surface opposed to the first surface, said compression plate compressing the object between said imaging stage (19) and itself;
an ultrasonic probe (16) provided to maintain acoustic connection with the second surface of said compression plate (13), for transmitting ultrasonic waves according to drive signals and receiving ultrasonic echoes generated when the transmitted ultrasonic waves are reflected by the object to output reception signals;
ultrasonic imagingmeans (42-46) for supplying the drive signals to said ultrasonic probe (16) and generating image data based on the reception signals outputted from said ultrasonic probe (16); and
control means (41) for controlling said ultrasonic imaging means (42-46) according to (i) information based on a material and/or a thickness of said compression plate (13), (ii) information based on a reception signal or an image signal from the object at a predetermined position, (iii) a detection result of an ultrasonic detector (100) provided in a position facing the first surface of said compression plate (13) in said imaging stage (19), for detecting ultrasonic waves transmitted from said ultrasonic probe (16) and passing through the object, or (iv) a detection result of an ultrasonic detector (100) provided to maintain acoustic connection with said compression plate (13), for detecting ultrasonic waves transmitted from said ultrasonic probe (16) and propagating in said compression plate (13), to adjust levels of the drive signals to be supplied to said ultrasonic probe (16).

2. The medical imaging apparatus according to claim 1, further comprising:
a storage unit (47) for storing correction amounts of ultrasonic outputs corresponding to attenuation amounts of ultrasonic waves measured or calculated based on materials and/or thicknesses of respective compression plates (13) according to types of compression plates (13); and
detectingmeans (14a) for detecting a type of compression plate (13) that has been actually attached;
wherein said control means (41) searches said storage unit (47) based on the type of compression plate (13) detected by said detecting means (14a) to load correction amount corresponding to the detected type of compression plate (13), and controls said ultrasonic imaging means (42-46) according to the loaded correction amount to adjust the levels of the drive signals to be supplied to the ultrasonic probe (16).

3. The medical imaging apparatus according to claim 1,
wherein said information based on a reception signal or an image signal from the object at a predetermined position represents an S/N ratio of an ultrasonic image of the object in the predetermined position.

4. The medical imaging apparatus according to claim 1 or 3, wherein said predetermined position is determined according to a distance from said compression plate (13).

5. The medical imaging apparatus according to claim 1 or 3, wherein said predetermined position is determined according to a thickness of the object compressed by said compression plate (13).
